Europäisches Patentamt

European Patent Office

Office européen des brevets

(11) Veröffentlichungsnummer: **0 094 593**
**A1**

(12) **EUROPÄISCHE PATENTANMELDUNG**

(21) Anmeldenummer: **83104581.0**

(22) Anmeldetag: **10.05.83**

(51) Int. Cl.³: **A 61 F 13/00**

(30) Priorität: **14.05.82 DE 3218322**

(43) Veröffentlichungstag der Anmeldung:
**23.11.83 Patentblatt 83/47**

(84) Benannte Vertragsstaaten:
**AT BE CH FR GB LI LU NL**

(71) Anmelder: **Rauscher & Co. Verbandstoff- und
Wattefabriken
Johann Schorsch Gasse 4
A-1141 Wien(AT)**

(72) Erfinder: **Waldner, Wilhelm
Ausgasse 9
A-2525 Schönau/Triesting(AT)**

(74) Vertreter: **Patentanwälte Leinweber & Zimmermann
Rosental 7/II Aufg.
D-8000 München 2(DE)**

(54) **Steril verpackter Wundschnellverband (Momentverband).**

(57) In einem sterilen Wundschnellverband ist ein Wundkissen mit einer durchlässigen Polypropylen-Hüllschicht in für die Automatenfertigung geeigneter Weise durch Aufsiegeln mit einer Binde (12a) verbunden, die zur Vermeidung von Verschmutzungen der Wundseite (20a) des Wundkissens (10a) beim Öffnen des Wundschnellverbandes unmittelbar vor und hinter dem Wundkissen mit je einer Verdrillung (24a, 26a) um 180° versehen ist. BeimAufziehen des Wundschnellverbandes und Spannen der Binde (12a) schlägt damit die bis dahin nach innen weisende und damit geschützte Wundseite selbsttätig in die Auflegelage um.

FIG.4

# LEINWEBER &
# ZIMMERMANN
0094593

## PATENTANWÄLTE

Dipl.-Ing. H. Leinweber (1930-76)
Dipl.-Ing. Heinz Zimmermann
Dipl.-Ing. A. Gf. v. Wengersky

Rosental 7 · D-8000 München 2
2. Aufgang (Kustermann-Passage)
Telefon (089) 2603989
Telex 528191 lepat d
Telegr.-Adr. Leinpat München

den   10 .5.1983

Unser Zeichen  WY

Rauscher & Co., Verbandstoff- und Wattefabriken

A-1147 Wien

## Steril verpackter Wundschnellverband (Momentverband)

Die Erfindung betrifft einen steril verpackten Wundschnellverband (Momentverband) bestehend aus einem Wundkissen
und einer Binde, an der das Wundkissen in der Nähe des einen
Bindenendes befestigt ist, wobei die Binde in der sterilen Verpackung in Form eines Wickels vorliegt, an dessen Außenumfang
das Wundkissen mit dem Bindenende zu liegen kommt, an dem es
befestigt ist.

Bekannte Wundschnellverbände dieser Art bestehen aus
einem gewebten Gazestreifen mit aufgenähtem Wundkissen aus
Zellstoff. In der sterilen Verpackung liegt üblicherweise
ein aus der Binde gebildeter Wickel neben dem Wundkissen.
Derartige Schnellverbände,sind umständlich in der Herstellung:

Das Aufnähen des Wundkissens läßt sich kaum automatisieren. Auch das Falten des freien Bindenendes ist ein schwer zu automatisierender Vorgang. Ein weiterer Nachteil besteht darin, daß bei diesem Aufbau eines Wundschnellverbandes häufig die Wundseite des Wundkissens beim Auflegen durch die verschmutzten Hände (Arbeitsunfall, Autounfall) selbst verschmutzt wird und nicht steril bleibt. Das erschwert durch Wundinfizierung den Heilungsvorgang. Nachteilig ist schließlich noch, daß beim bekannten Wundschnellverband das Wundkissen bei längerem Verbleiben auf der Wunde leicht mit dieser verklebt. Das ist unerwünscht.

Es ist deshalb Aufgabe der Erfindung, einen Wundschnellverband vorzuschlagen, der durch seinen Aufbau einer Verschmutzung der Wundseite des Wundkissens beim Anlegen des Verbandes entgegenwirkt und Verkleben mit der Wunde vermeidet, dabei aber ein kontinuierliches und weitgehend automatisierbares Herstellungsverfahren ermöglicht. Diese Aufgabe wird durch die im Anspruch 1 gekennzeichnete Erfindung gelöst. Zweckmäßige Ausgestaltungen sind in den Unteransprüchen angegeben.

Man erkennt, daß hier die Binde des Wundschnellverbandes nicht einfach zu einem zylindrischen oder sonst auf zweckmäßige Weise geformten Wickel aufgerollt wird. Statt dessen wird die Binde vor und hinter dem Wundkissen um 180° verdreht. Damit kann erreicht werden, daß im verpackten Wundschnellverband die Wundseite des Wundkissens nach innen weist und dadurch beim Öffnen mit verschmutzten Händen vor Verschmutzungen gesichert wird. Besonders zweckmäßig ist es, wenn sich im verpackten Zustand die Wundseite des Wundkissens auf der Außenseite des Bindenwickels abstützt und radial nach innen auf dessen Zentrum zu weist. Dennoch genügt es in Anbetracht der beiden 180°-Verdrillungen der Binde vor und hinter dem Wundkissen nach dem Aufreißen der Verpackung und dem Ergreifen des Wickels mit der einen und des freien Bindenendes mit der anderen Hand beide Teile kurz auseinanderzuziehen, wie das beim Spannen vor dem Auflegen eines Wundschnellverbandes jedenfalls notwendig ist. Das Wundkissen schlägt dann aufgrund der jetzt eintretenden Öffnung der beiden Verdrillungen unter den Zugkräften um und kann sauber unmittelbar auf die Wunde aufgelegt werden. Die beiden 180°-Verdrillungen können ohne Schwierigkeiten in den automatischen Herstellungsvorgang des Wundschnellverbandes einbezogen werden.

Besonders günstig ist es, wenn das Wundkissen zumindest wundseitig, zweckmäßig aber ringsum eine Polypropylen-Hüllschicht aufweist. Es kann dann nämlich durch Aufsiegeln unter Einwirkung von Wärme und/oder Druck an der Binde befestigt werden. Das ist wiederum ein einfacher, automatengerechter Vorgang und insoweit dem Aufnähen des Wundkissens auf der Binde überlegen.

Als Zusatzeffekt wird durch die Eigenschaften der Polypropylen-Hüllschicht ein Verkleben mit der Wunde vermieden. Die gegebenenfalls poröse, zweckmäßig gitterartig aufgebaute Polypropylen-Hüllschicht erlaubt aber den Sektretabtransport von der Wunde durch die Schicht hindurch in das Innere des Wundkissens. Dieser Abtransport wird noch begünstigt, wenn die an die Polypropylen-Hüllschicht anschließende Schicht eine Vliesschicht ist, die mit der Polypropylen-Hüllschicht durch das Aufsiegeln verbunden ist und aufgrund ihrer hydrophilen Eigenschaften den Sektretabtransport in die Zellstofffüllung im Inneren des Wundkissens begünstigt.

Im Querschnitt gesehen wird zweckmäßig die Außenseite des Wundkissens durch einen Kreisbogen begrenzt. Das erbringt über der Wunde eine ausreichende Zellstoffauflagendicke für Sektretaufnahme und Abpolsterung. Gleichzeitig wird durch die Querschnittsform des Wundkissens in Form eines Kreisabschnittes eine kontinuierliche Verminderung der Wundkissenstärke zu den Wundkissenrändern hin erreicht, die wiederum den kontinuierlichen Übergang in die Binde begünstigt, mit der das Wundkissen durch Aufsiegeln verbunden ist. Die Vorformung des Wundkissens kann auch so erfolgen, daß nicht nur seine Außenseite, sondern auch seine Innenseite durch einen Kreisbogen begrenzt ist. Der letztere weist dann einen größeren Durchmesser auf. Es entsteht also ein auch wundseitig sphärisch leicht vorgewölbtes Wundkissen. Dies ist beim Verbinden von Körperextremitäten und den meist gewölbten Körperflächen günstig. Es erbringt zusätzlich eine bessere Abstützung des Wundkissens auf der Außenseite des Bindenwickels. Da diese ebenfalls sphärisch geformt ist, wird das Wundkissen im verpackten Zustand einer geringeren Deformation unterworfen.

Weitere Einzelheiten, Vorteile und Merkmale ergeben sich aus der folgenden Beschreibung anhand der Figuren, auf die wegen der Offenbarung aller im folgenden nicht näher erläuterten Einzelheiten ausdrücklich verwiesen wird. Es zeigen:

Fig. 1    einen bekannten Wundschnellverband,

Fig. 2    den Wundschnellverband nach Fig. 1 in der Hand des Helfers nach Öffnen der Verpackung,

Fig. 3    eine Fig. 2 entsprechende Darstellung einer Ausführungsform der Erfindung,

Fig. 4   eine perspektivische Ansicht eines vom
Bandende her schon teilweise geöffneten
Wundschnellverbandes nach der Erfindung
zur Erläuterung des Wickelprinzips nach
der Erfindung, und

Fig. 5   eine Fig. 1 entsprechende Darstellung
eines Wundschnellverbandes nach der Erfindung.

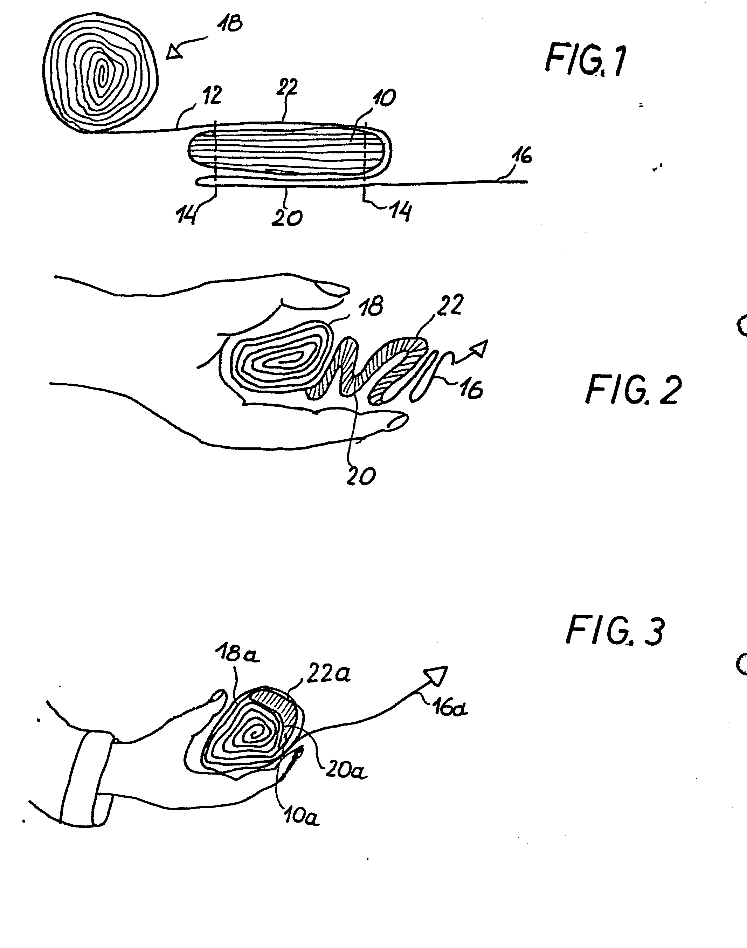

Fig. 1 zeigt einen herkömmlichen Wundschnellverband (Momentverband), bestehend aus einem Wundkissen 10
und einer Binde 12. Das Wundkissen 10 besteht aus Zellstoff, die Binde 12 aus einem gewebten Gazestreifen. Das
Wundkissen 10 ist durch Nähte 14 an der Binde 12 befestigt. Die Binde 12 hat ein freies Bandende 16. In
Nähe dieses freien Bandendes ist das Wundkissen 10 mit
der Binde 12 verbunden. Das freie Bandende 16 ist deshalb kurz. Das andere, lange Bandende ist zu einem Bindenwickel 18 aufgewickelt. Mit Hilfe des Bindenwickels kann
durch wiederholtes Umschlingen des zu verbindenden
Körperteils das Wundkissen 10 auf der Wunde befestigt
werden. Diese Art des Auflegens und Befestigens ist
allen Wundschnellverbänden gemeinsam.

In der Fig. 1 ist der Wundschnellverband zur Verdeutlichung in einer idealisierten Lage gezeigt. Dabei
ist angenommen, daß auf übliche Weise durch Erfassen des
freien Bandendes 16 und des Bindenwickels 18 und Auseinanderziehen dieser beiden Teile zur Straffung des
dazwischenliegenden Teils das Wundkissen 10 zum Auflegen
auf die Wunde bereitgestellt wird. Die Wundseite 20 des
Wundkissens 10 liegt also in Fig. 1 unten, die wundabgewandte Außenseite 22 oben. Es ist leicht zu erkennen, daß
bei auf den Umfang des Bindenwickels 18 aufgewickeltem Wundkissen 10 dessen Außenseite 22 innen, die als Wundauflage bestimmte Wundseite 20 jedoch außen zu liegen
kommt.

Fig. 2 zeigt den bekannten Wundschnellverband
nach Fig. 1 nach dem Aufreißen seiner sterilen Verpackung
in der Hand des Helfers. Der Bindenwickel 18 liegt in der
Handmulde. Das freie Bandende 16 der Binde wird in Richtung des Pfeils ausgezogen. Das gefaltete Wundkissen 10
wird dabei gestrafft. Seine spätere Außenseite 22 weist
dabei nach oben, von den Fingern der Hand weg, während
seine Wundseite 20 den Fingern der Hand zugekehrt ist.
Bei verschmutzten Händen läßt es sich dabei kaum vermeiden, daß zumindest die Wundseite 20, häufig aber auch
die Außenseite 22 des Wundkissens ebenfalls beschmutzt
wird. Bei Arbeits-, Berg-, Autounfällen oder dergleichen
sind aber die verschmutzten Hände des Helfers oder des
sich selbst den Verband anlegenden Patienten kaum zu
vermeiden. Die Verschmutzung der Wundseite 20 des Wundkissens 10 führt aber häufig zu Wundinfektionen, die
den späteren Heilungsprozeß beeinträchtigen.

Fig. 3 zeigt nun den erfindungsgemäßen Bindenwickel 18a mit noch eingewickeltem Wundkissen 10a und
dem freien Bandende 16a, das wieder in Pfeilrichtung
auszuziehen ist. Man erkennt schon hier, daß das Wundkissen 10a im Querschnitt die Form eines Kreisabschnittes
hat. Dieser ist zumindest auf der Außenseite 22a durch
einen Kreisbogen begrenzt. Die Wundseite 20a kann eben
oder ebenfalls durch einen Kreisbogen begrenzt sein. Der
Radius dieses Kreisbogens ist im letzteren Falle größer
als der des die Außenseite 22 definierenden Kreisbogens.
Man erkennt aus Fig. 3 insbesondere, daß hier die Wundseite 20a des Wundkissens 10a zum Inneren des Bindenwickels 18a hinweist, während die Außenseite 22a radial
außen liegt. Beim Ausziehen des freien Bandendes 16a gleitet deshalb lediglich die Außenseite 22a des Bindenwickels 18a über die möglicherweise verschmutzte Handfläche. Eine Berührung zwischen der steril zu erhaltenden Wundseite 20a des Wundkissens 10a und der Handfläche wird aber zuverlässig vermieden. Dennoch ist das Ausspannen und Auflegen des Wundkissens auf die Wunde ohne Schwierigkeiten möglich, wie sogleich erläutert werden soll.

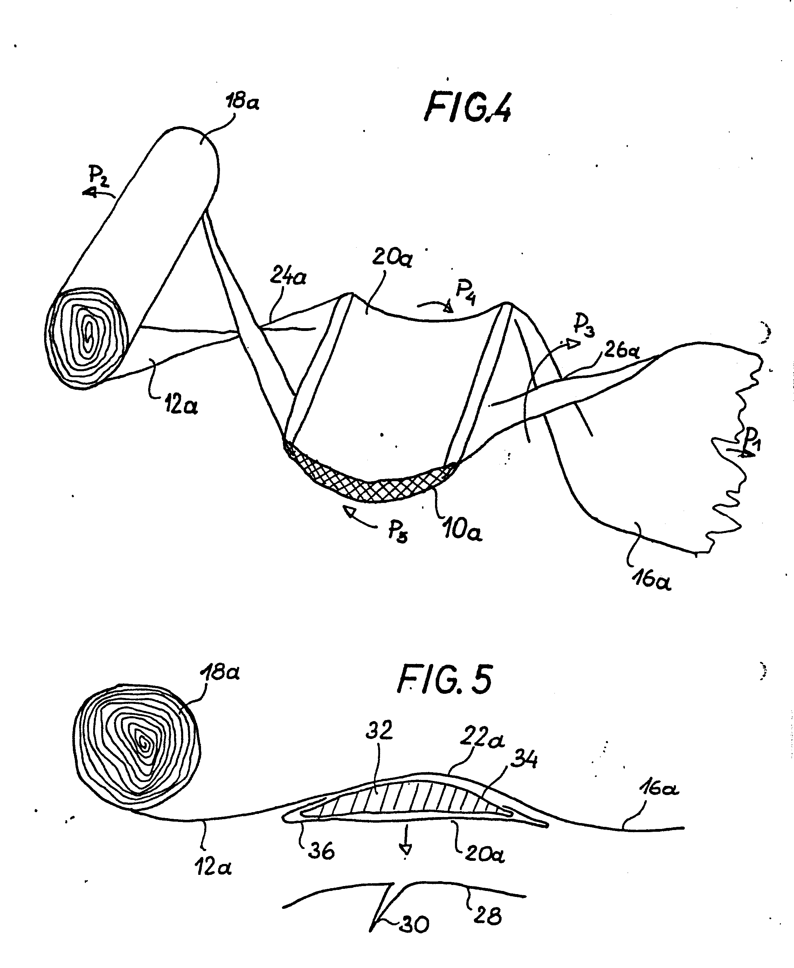

Fig. 4 zeigt hierfür die Lage des Wundkissens 10a nach dem Ausziehen des freien Bandendes 16a. Man erkennt, daß die Binde 12a unmittelbar vor und hinter dem Wundkissen 10a zwei Verdrillungen 24a und 26a aufweist, in denen die Binde 12a um 180° verdreht ist.

In diesem Zustand wird nun auf übliche Weise eine Spannkraft ausgeübt. Dafür wird das freie Bandende 16a in Richtung des Pfeils P1 und der verbliebene Binden- wickel 18a in Richtung des Pfeils P2 , also in Gegenrichtung bewegt. Durch diese Spannkräfte werden die jetzt nicht mehr am Bandwickelumfang festgelegten Verdrillungen 24a und 26a aufgezogen und haben das Bestreben, sich zu längen. Dies ist nur durch eine Bewegung in Richtung des Pfeils P3 möglich. Dadurch wird aber dem Wundkissen ein Umschlag aufgezwungen, wie er durch die Pfeile P4 und P5 angedeutet ist. Es bewegt sich somit um eine in Bandlängsrichtung durch die Verdrillungen 24a und 26a gelegte Achse und schlägt um 180° um. Die bisher oben oder bezüglich des Bandwickels 18a innen liegende sterile Wundseite 20a kommt dabei unten, also über der Wunde zu liegen, die bisher unten liegende Außenseite 22a oben. Eine Berührung des Wundkissens für diesen Vorgang ist nicht erforderlich. Es genügt das übliche Ausspannen, um diesen Vorgang selbsttätig auszulösen.

Fig. 5 zeigt das Ergebnis dieses Vorgangs. Die Verdrillungen 24a und 26a sind verschwunden, die Außenseite 22a des Wundkissens liegt, wie in Fig. 1, nunmehr oben, während die steril gebliebene Wundseite 20a über der Hautoberfläche 28 mit der Wunde 30 zu liegen kommt und in Pfeilrichtung auf diese aufgelegt werden kann. Anschließend erfolgt auf übliche Weise durch Umwickeln mit der Binde 12a eine Fixierung auf der Wunde 30.

In den Figuren 4 und 5 ist nochmals deutlich zu erkennen, daß die Außenseite 22a des Wundkissens 10a eine zylindrische Fläche bildet. Die Wundseite 20a kann je nach Bedarf ebenfalls leicht zylindrisch vorgeprägt oder auch eben sein. In beiden Fällen paßt sie sich unter den durch die Binde 12a ausgeübten Zugkräften der Körperoberfläche gut an.

Das Wundkissen besteht aus einer Zellstoffüllung 32, die in eine Vliestasche 34 eingebracht ist. Selbstverständlich kann auch jede andere Form der Vliesumhüllung der Zellstoffüllung 32 gewählt werden. Das ganze Wundkissen 10a ist dann von einer Polypropylenhüllschicht 36 umgeben. Diese verklebt nicht mit der Wunde, läßt aber einen Abtransport des Wundsekretes unter der Saugwirkung der Vliesschicht in die Zellstoffüllung 32 hinein zu. Weiter erlaubt die Polypropylen-Hüllschicht eine Verbindung mit dem die Binde 12a bildenden Gazegewebe durch Aufsiegeln. Der Wundschnellverband kann deshalb aufgrund seines erläuterten Aufbaus insgesamt einfach und billig, sowie automatengerecht hergestellt werden.

## Bezugszeichenliste

### Steril verpackter Wundschnellverband (Momentverband)

10  Wundkissen (Stand der Technik)

10a Wundkissen (Erfindung)

12, 12a Binde

14  Nähte

16, 16a  Bandende

18, 18a Bindenwickel

20, 20a Wundseite (von 10, 10a)

22, 22a Außenseite (von 10, 10a)

24a Verdrillung

26a Verdrillung

28  Hautoberfläche

30  Wunde

32  Zellstoffüllung

34  Vliestasche

36  Polypropylen-Hüllschicht

P 1 bis P 5  Pfeile

Patentansprüche

1. Steril verpackter Wundschnellverband (Momentverband),bestehend aus einem Wundkissen und einer Binde, an der das Wundkissen in der Nähe des einen Bindenendes befestigt ist, wobei die Binde in der sterilen Verpackung in Form eines Wickels vorliegt, an dessen Außenumfang das Wundkissen mit dem Bindenende zu liegen kommt, an dem es befestigt ist, dadurch gekennzeichnet, daß die Binde (12a) im verpackten Wundschnellverband vor und hinter dem Wundkissen (10a) um 180° verdreht ist.

2. Wundschnellverband nach Anspruch 1, dadurch gekennzeichnet, daß sich im verpackten Wundschnellverband das Wundkissen (10a) mit seiner radial nach innen weisenden Wundseite (20a) auf der Außenseite des Bindenwickels (18a) abstützt.

3. Wundschnellverband nach Anspruch 1 oder 2, dadurch gekennzeichnet, daß das Wundkissen (10a) wundseitig eine Polypropylen-Hüllschicht (36) aufweist.

4. Wundschnellverband nach Anspruch 3, dadurch gekennzeichnet, daß die Polypropylen-Hüllschicht (36) das Wundkissen (10a) vollständig umhüllt.

5. Wundschnellverband nach Anspruch 3 oder 4, dadurch gekennzeichnet, daß die Hüllschicht (36) des Wundkissens (10a) an der vorzugsweise aus Gaze bestehenden Binde (12a) durchAufsiegeln befestigt ist.

6. Wundschnellverband nach einem der Ansprüche 3 bis 5, dadurch gekennzeichnet, daß das Wundkissen (10a) aus einer Zellstoffüllung (32) in einer Vliestasche (34) besteht, die selbst wieder von der Polypropylen-Hüllschicht (36) umgeben ist.

7. Wundschnellverband nach einem der Ansprüche 1 bis 6, dadurch gekennzeichnet, daß die Außenseite (22a) des Wundkissens (10a) im Querschnitt durch einen Kreisbogen begrenzt ist.

8. Wundschnellverband nachAnspruch 7, dadurch gekennzeichnet, daß die Innenseite (Wundseite 20a) des Wundkissens (10a) im Querschnitt durch einen Kreisbogen begrenzt ist, dessen Radius größer ist als der die Außenseite bildende Kreisbogen.

0094593

*FIG. 1*

*FIG. 2*

*FIG. 3*

# FIG.4

# FIG.5

**Europäisches Patentamt**

**EUROPÄISCHER RECHERCHENBERICHT**

**0094593**
Nummer der Anmeldung

EP 83 10 4581

## EINSCHLÄGIGE DOKUMENTE

| Kategorie | Kennzeichnung des Dokuments mit Angabe. soweit erforderlich. der maßgeblichen Teile | Betrifft Anspruch | KLASSIFIKATION DER ANMELDUNG (Int. Cl. ³) |
|---|---|---|---|
| A | US-A-3 421 502 (G.P. ST.CLAIR) <br> * Insgesamt * | 1 | A 61 F 13/00 |
| A | US-A-4 297 996 (E. URIZA) <br> * Figur 4; Spalte 3, Zeilen 21-37* | 1 | |
| A | DE-A-2 515 786 (A. LAERDAL) <br> * Insgesamt * | 1 | |
| | ----- | | |

RECHERCHIERTE SACHGEBIETE (Int. Cl. ³)

A 61 F

Der vorliegende Recherchenbericht wurde für alle Patentansprüche erstellt.

| Recherchenort | Abschlußdatum der Recherche | Prüfer |
|---|---|---|
| DEN HAAG | 25-08-1983 | LEVENBACH J.H. |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie. übereinstimmendes Dokument

EPA Form 1503 03 82